# EUROPÄISCHE PATENTSCHRIFT

(11) **EP 3 185 945 B1**
(45) Veröffentlichungstag und Bekanntmachung des Hinweises auf die Patenterteilung: **08.05.2019**
(21) Anmeldenummer: 16785381.1
(22) Anmeldetag: 05.10.2016
(51) Int. Cl.: A61M 39/28, A61M 5/142

(54) **MEDIZINISCHE PUMPE MIT SCHLAUCHKLEMMENAUFNAHME, SCHLAUCHKLEMME SOWIE SYSTEM AUS BEIDEN**
MEDICAL PUMP WITH TUBE CLAMP HOLDER, TUBE CLAMP AND SYSTEM OF BOTH
POMPE MÉDICALE AVEC SUPPORT DE PINCE DE TUBES, PINCE DE TUBE ET SYSTÈME DES DEUX

(30) Priorität: 14.10.2015 DE 102015117493
(43) Veröffentlichungstag der Anmeldung: 05.07.2017
(73) Patentinhaber: B. Braun Melsungen AG, 34212 Melsungen (DE)
(72) Erfinder: STEGER, Jürgen, 34327 Körle (DE); HEITMEIER, Rolf, 34225 Baunatal (DE); KATERKAMP, Andreas, 34212 Melsungen (DE); ERLEN, Christoph, 34132 Kassel (DE); KRAMER, Matthias, 34212 Melsungen (DE)
(74) Vertreter: Winter, Brandl, Fürniss, Hübner, Röss, Kaiser, Polte - Partnerschaft mbB
(86) Internationale Anmeldenummer: PCT/EP2016/073791
(87) Internationale Veröffentlichungsnummer: WO 2017/063930

(56) Entgegenhaltungen:
- EP-A1- 2 583 716
- EP-A1- 2 921 188
- WO-A2-2011/119425
- US-A1- 2014 216 557

## Beschreibung

### Technisches Gebiet

Die vorliegende Erfindung bezieht sich auf medizinische Pumpen gemäß dem Oberbegriff des Anspruchs 1, insbesondere auf eine medizinische Pumpe, welche einen Anschluss für einen Schlauch aufweist, in dessen Inneren ein Medium mittels der Pumpe gefördert werden kann, und welche eine Aufnahme aufweist, in der eine von der Pumpe separate Schlauchklemme aufgenommen bzw. werkzeuglos angebracht werden kann.

Des Weiteren betrifft die vorliegende Erfindung eine Schlauchklemme gemäß den Ansprüchen 12 oder 13, welche an einer erfindungsgemäßen Pumpe anbringbar ist und mittels derer ein an der Pumpe angebrachter Schlauch so abklemmbar ist, dass durch das Innere des Schlauchs kein Medium fließen bzw. strömen kann.

Schließlich bezieht sich die vorliegende Erfindung auch auf ein System gemäß Anspruch 16 bestehend aus einer erfindungsgemäßen Pumpe und einer erfindungsgemäßen Schlauchklemme.

### Stand der Technik

In der Infusionstechnik werden Überleitsysteme zum Fördern von Medikamenten in den Körper des Patienten eingesetzt. Diese Überleitsysteme bewerkstelligen die Überleitung von Medikamente zumeist mittels Schläuchen bzw. Schlauchsets und besitzen üblicherweise Absperrvorrichtungen, z.B. Rollenklemmen, zur Unterbrechung der Förderung.

Bei Anwendungen mit Anforderung an eine hohe Dosiergenauigkeit sind Infusionen allein mittels Schwerkraft unzureichend und werden deshalb Infusionspumpen eingesetzt, wobei insbesondere lebenserhaltende Medikamente mittels Infusionspumpen verabreicht werden.

Bei einem Infusionspumpensystem müssen Vorkehrungen getroffen werden, dass es beim Hantieren eines an einer Infusionspumpe angebrachten Schlauchsets oder beim Öffnen der Infusionspumpe zum Unterbrechen der Medikamentenförderung zu keiner für einen Patienten lebensbedrohenden Free-Flow-Situation kommt, in welcher dem Patienten das Medikament unkontrolliert bzw. undosiert vor allem mittels Schwerkraft zugeführt wird.

Zum einen sind beispielsweise aus EP 2 716 312 A1 oder US 2013/0253442 A1 Infusionspumpen bekannt, welche eine pumpenseitige Sicherheitsklemme aufweisen, die verhindert, dass es beim Öffnen der Infusionspumpe trotz nicht geschlossener Rollenklemme zu einer Free-Flow-Situation kommt.

Zum anderen sind beispielsweise aus EP 2 583 716 A1 oder EP 2 780 070 A1 auch Infusionssysteme bekannt, welche mit einer sogenannten setseitigen Free-Flow-Klemme ausgerüstet sind. Diese Klemme befindet sich unmittelbar am Einmalartikel, beispielsweise an dem Schlauch bzw. Schlauchset und verschließt diesen Einmalartikel mit dem Öffnen einer Pumpenklappe.

Ein Nachteil dieses Standes der Technik ist es, dass sich Anwender auf die Funktion der setseitigen Free-Flow-Klemme verlassen und nun regelmäßig die Rollenklemme nicht mehr schließen.

Um eine Patientengefährdung zu minimieren, ist es vor dem Hintergrund dieses Stands der Technik also notwendig, sowohl eine pumpenseitige Sicherheitsklemme, als auch eine setseitige Free-Flow-Klemme vorzusehen.

Eine medizinische Pumpe gemäß dem Oberbegriff des Anspruchs 1 ist aus der WO 2011/119425 A2 bekannt. Weiterer für die vorliegende Erfindung relevanter Stand der Technik findet sich in der EP 2 921 188 A1 und der US 2014/216557 A1.

### Offenbarung der Erfindung

Aufgabe der vorliegenden Erfindung ist es deshalb, eine medizinische Pumpe und/oder eine Schlauchklemme bereitzustellen, mit der die bisher gewohnte Patientensicherheit, insbesondere während einer Infusion, verbessert oder weniger aufwendig gewährleistet werden kann.

Diese Aufgabe wird durch Merkmale der Ansprüche 1, 12, 13 und 16 gelöst. Vorteilhafte Weiterbildungen sind Gegenstand der Unteransprüche.

Eine erfindungsgemäße medizinische Pumpe, insbesondere Infusionspumpe, zum Fördern eines Mediums, weist eine Aufnahme für eine von der Pumpe separate, an einem zumindest abschnittsweise flexiblen Schlauch angeordnete Schlauchklemme auf. Zwei relativ zueinander bewegliche Klemmabschnitte der Schlauchklemme sind im Bezug aufeinander in eine geschlossene und in eine geöffnete Relativposition bringbar. In der geschlossenen Relativposition klemmen die Klemmabschnitte den zwischen sich angeordneten Schlauch so ab, dass durch das Innere des Schlauchs kein Medium fließen kann. In der geöffneten Relativposition klemmen die Klemmabschnitte den zwischen sich angeordneten Schlauch nicht ab, so dass durch das Innere des Schlauchs ein Medium bzw. ein Medikament und/oder eine Körperflüssigkeit wie Blut fließen kann.

Die erfindungsgemäße Pumpe zeichnet sich dadurch aus, dass an ihr, insbesondere in der Aufnahme für die Schlauchklemme, ein Sensor vorgesehen ist, welcher bei der in der Aufnahme aufgenommenen Schlauchklemme zumindest erfasst, ob sich deren Klemmabschnitte in der geschlossenen oder der geöffneten Relativposition befinden.

Der Sensor ist also in der Lage, zumindest zwei Messwerte zu erfassen, wovon ein Messwert eindeutig der geschlossenen Relativposition der Klemmabschnitte der in der Aufnahme aufgenommenen Schlauchklemme zugeordnet ist und der andere Messwert eindeutig der geöffneten Relativposition der Klemmabschnitte der in der Aufnahme aufgenommenen Schlauchklemme zugeordnet ist. Befindet sich keine Schlauchklemme in der Aufnahme, erfasste der Sensor den gleichen Messwert wie bei der geöffneten Relativposition der Klemmabschnitte der in der Aufnahme aufgenommenen Schlauchklemme. Optional könnte Sensor auch derart ausgebildet sein, dass dieser bei einer nicht in die Aufnahme eingelegten Schlauchklemme einen eindeutigen dritten Messwert erfasst.

Die Aufnahme für die Schlauchklemme ist derart ausgebildet, dass die Schlauchklemme bzw. zumindest einer der zwei Klemmabschnitte an der Pumpe werkzeuglos angebracht und von der Pumpe zerstörungsfrei entfernt werden kann. Insbesondere weist die Aufnahme Hinterschneidungen auf, hinter welchen Abschnitte der Schlauchklemme eingeklemmt werden können.

Der Sensor kann derart ausgebildet sein, dass er die geschlossene und die geöffnete Relativposition zweier relativ zueinander verschwenkbarer Klemmabschnitte der in der Aufnahme aufgenommenen Schlauchklemme detektieren kann. Alternativ oder zusätzlich kann der Sensor auch die geschlossene und die geöffnete Relativposition zweier relativ zueinander verschiebbarer Klemmabschnitte oder zweier relativ zueinander verschwenk- und verschiebbarer Klemmabschnitte der in der Aufnahme aufgenommenen Schlauchklemme detektieren.

Der Sensor kann ein Tastsensor sein, welcher auf Kontakt mit der Schlauchklemme bzw. mit einem Teil der Schlauchklemme anspricht. Alternativ kann der Sensor auch optische, elektrische oder magnetische Effekte ausnutzen, um die geschlossene Relativposition zu detektieren.

Vorteil der vorliegenden Erfindung ist es, dass Fehler in der setseitigen Schlauchklemme bzw. Free-Flow-Klemme erkannt werden können, bevor es zu einer Patientengefährdung kommen kann, und der Anwender entsprechend reagieren kann.

Aufgabe der Erfindung nach Anspruch 1 ist es Fehler zu erkennen, die während der Herstellung der Schlauchklemme (z.B. Verzug durch Abkühlung) und/oder erst nach dem in-Verkehr-Bringen (z.B. mechanische Überlastung durch Fehlbedienung) der Schlauchklemme entstehen.

In vorteilhafter Weise müssen für die erfindungsgemäße Pumpe geeignete Schlauchklemmen nicht zwangsläufig die Anforderung der Erstfehlersicherheit erfüllen und können die Qualitätsanforderungen einer erfindungsgemäßen Klemme auf das Niveau einer üblichen Rollenklemme reduziert werden. Hierdurch können Herstellkosten, beispielsweise durch Verzicht auf 100-Prozent-Dichtigkeit-Prüfungen, eingespart werden.

Die Schlauchklemme nimmt als Sicherheitsklemme während des Normalbetriebs im Wesentlichen zwei Stellungen ein: eine geschlossene Stellung, in welcher der Schlauch abgeklemmt ist, und eine geöffnete Stellung, in welcher der Schlauch nicht abgeklemmt ist. Werden beide Stellungen durch den Sensor explizit erfasst, wird eine redundante Überprüfungslogik umgesetzt, bei welcher der Sensor sowohl während des geschlossenen Zustands der Schlauchklemme, als auch im geöffneten Zustand der Schlauchklemme, stets ein Signal ausgibt.

Die medizinische Pumpe weist eine elektronische Datenverarbeitungseinheit auf, welche die zum Einsatz der Schlauchklemme erforderliche strukturelle und/oder funktionelle Integrität der in der Aufnahme aufgenommenen Schlauchklemme dadurch erfasst, dass sie den zeitlichen Ist-Verlauf der durch den Sensor durchgeführten Messung beim Übergang der Klemmabschnitte von der geschlossenen zur geöffneten oder von der geöffneten zur geschlossenen Relativposition mit einem zeitlichen Soll-Verlauf der Messung vergleicht. Treten bei diesem Vergleich des tatsächlichen dynamischen Verhaltens der Schlauchklemme mit dem zu erwartenden dynamischen Verhaltens der Schlauchklemme Abweichungen auf, kann durch die Datenverarbeitungseinheit ein Alarmsignal ausgelöst werden.

Die Überwachung des dynamischen Verhaltens der Klemmabschnitte ist vorteilhaft, weil dadurch sich schleichend anbahnende Schäden an der Schlauchklemme früher erkannt werden können.

An der medizinischen Pumpe kann ein Aktor vorgesehen sein, welcher die Klemmabschnitte der in der Aufnahme aufgenommenen Schlauchklemme von der geschlossenen in die geöffnete und/oder von der geöffneten in die geschlossene Relativposition bringen kann. Der Aktor ist insbesondere ein durch einen Schrittmotor bewegliches Element, welches zum Öffnen der geschlossenen Schlauchklemme zwischen die Klemmabschnitte eindringt und somit den Abstand zwischen den Klemmabschnitte vergrößert. Alternativ kann der Aktor zum Öffnen einer wippenähnlichen oder wäscheklammerähnlichen Schlauchklemme lediglich auf einen der Klemmabschnitte einwirken. Auch sich kann der Aktor aus zwei Elementen zusammen setzen, wobei ein Element zum Bewegen eines der Klemmabschnitte in eine Richtung dient und das andere Element zum Bewegen des gleichen Klemmabschnitts in eine entgegen gesetzte Richtung dient.

Der Vorteil eines steuerbaren Aktors ist, dass das Öffnen der Schlauchklemme stets auf gleiche Art und Weise ausgeführt werden kann und somit Schäden durch unsachgemäßes Öffnen besser vermieden werden können.

Die medizinische Pumpe kann in vorteilhafter Weise eine Pumpenklappe aufweisen, welche in einer geschlossenen Stellung die Aufnahme derart abdeckt, dass die Schlauchklemme nicht aus der Aufnahme entnehmbar oder nicht in die Aufnahme einbringbar ist, und in einer geöffneten Stellung die Aufnahme derart freigibt, dass die Schlauchklemme aus der Aufnahme entnehmbar oder in die Aufnahme einbringbar ist.

Durch die Pumpenklappe kann die vom Sensor durchgeführte Messung der Relativposition der Klemmabschnitte der in der Aufnahme aufgenommenen Schlauchklemme von externen Störgrößen abgeschirmt werden, womit die Qualität der Messung gewährleistet werden kann. Auch Umweltfaktoren, welche die Schlauchklemme oder den Sensor schädigen, können durch die Pumpenklappe abgeschirmt werden. Optional ist es auch möglich, die Pumpenklappe derart auszubilden, dass durch Bewegen der Pumpenklappe der Zustand der Schlauchklemme beeinflusst wird. Beispielsweise kann sie derart ausgebildet sein, dass die Schlauchklemme beim Schließen der Pumpenklappe geöffnet wird.

In vorteilhafter Weise kann der Aktor die Klemmabschnitte der in der Aufnahme aufgenommenen Schlauchklemme bei geschlossener Stellung der Pumpenklappe von der geschlossenen in die geöffnete und/oder von der geöffneten in die geschlossene Relativposition bringen kann. Somit kann nicht nur die durch den Sensor durchgeführte Messung sondern auch kann auch der durch den Aktor durchgeführte Öffnungs- und Schließungsvorgang vor schädlichen Störgrößen abgeschirmt werden.

Die medizinische Pumpe kann derart ausgebildet sein, dass die geöffnete Relativposition der Klemmabschnitte der in der Aufnahme aufgenommenen Schlauchklemme durch den Aktor derart veränderlich ist, dass ein Öffnungsgrad des Inneren des Schlauchs und somit ein Volumenstrom des durch den Schlauch strömenden Mediums einstellbar ist. Der erfindungsgemäße Sensor kann auch derart ausgebildet sein, dass er mehrere geöffnete Relativpositionen der Klemmabschnitte der an der Aufnahme befindlichen Schlauchklemme detektieren kann, so dass mittels des Sensors der Öffnungsgrad des Inneren des Schlauchs und somit der Volumenstrom des durch den Schlauch strömenden Mediums erfassbar ist.

Wenn mittels des Sensors mehrere geöffnete Relativpositionen der Klemmabschnitte detektierbar sind, dann kann die erfindungsgemäße Schlauchklemme nicht nur die Sicherungsfunktion besser erfüllen, sondern kann diese auch der Dosierung des durch den Schlauch strömenden Mediums dienen. Die Verbesserung der Sicherungsfunktion ist darauf zurückzuführen, dass durch die Überwachung mehrerer von der Schlauchklemme einnehmbarer Zustände die Position der Klemmabschnitte bei einer Fehlfunktion genauer bestimmbar ist. Wird an der Pumpe der Aktor vorgesehen, welcher die Relativposition der Klemmabschnitte der Schlauchklemme ändern kann, und ist der Sensor in der Lage diese vom Aktor eingestellten Relativpositionen zu detektieren, dann ist eine Dosierung mittels der Schlauchklemme möglich und können sonst notwendige Ventile oder Rollenklemmen an der Pumpe weggelassen werden.

Der Sensor kann so ausgebildet sein, dass er die geschlossene Relativposition der beiden Klemmabschnitte erfassen, indem er die Position des ersten Klemmabschnitts detektiert. Die Aufnahme ist insbesondere so ausgebildet, dass in ihr ein zweiter der beiden Klemmabschnitte fixiert werden kann. Diese Verbindung kann mittels einer Klemmverbindung umgesetzt werden. Die Aufnahme und der Sensor können relativ zueinander so positioniert sein, dass der Sensor nur dann das Signal für die geschlossene Relativposition der Klemmabschnitte ausgeben kann, wenn der zweite Klemmabschnitt mit der Aufnahme, insbesondere in einer vorbestimmten, einzig möglichen Art, verbunden ist.

Wird der Sensor derart ausgebildet, dass die geschlossene Relativposition der Klemmabschnitte unmittelbar mittels Messen des Abstands zwischen der beiden Klemmabschnitten erfasst wird, kann die Überwachung der Schlauchklemme auf effiziente Art umgesetzt werden.

Die Pumpe kann einen Grundkörper aufweisen, demgegenüber die Pumpenklappe relativ beweglich ist. Die Aufnahme kann an dem Grundkörper oder an der Pumpenklappe vorgesehen sein und der zweite Klemmabschnitt kann mittels der Pumpenklappe bewegbar sein.

Der Anschluss für den Schlauch kann unter der Pumpenklappe angeordnet, so dass zum An- und Abkoppeln des Schlauchs die Pumpenklappe geöffnet bzw. geschlossen werden muss. Insbesondere kann die Pumpenklappe so ausgeformt sein, dass durch Öffnen der Pumpenklappe die in der Aufnahme befindliche Schlauchklemme geschlossen wird und/oder durch Schließen der Pumpenklappe die in der Aufnahme befindliche Schlauchklemme geöffnet wird.

Durch Vorsehen einer auf die Schlauchklemme wirkenden Pumpenklappe kann in vorteilhafter Weise sichergestellt werden, dass bei der Entnahme des Schlauchs aus der Pumpe ein Schließen der Schlauchklemme zwangsläufig erfolgt oder dass ein einwandfreies Öffnen der Pumpe und eine Entnahme des Schlauchs nur dann möglich sind, wenn das Schließen der Schlauchklemme ohne Komplikationen erfolgt ist.

Der Sensor und die Aufnahme können beide am Grundkörper angeordnet sein. Alternativ wäre es auch möglich, beide an der Pumpenklappe anzuordnen. Auch könnten der Sensor und die Aufnahme auf die beiden Pumpenteile verteilt werden (d.h. Sensor am Grundkörper und Aufnahme an Pumpenklappe oder Aufnahme am Grundkörper und Sensor an der Pumpenklappe).

Die Pumpenklappe kann schwenkbar mit dem Grundkörper verbunden sein.

Der erfindungsgemäße Sensor kann ein Lichtsensor sein, welcher auf eine Unterbrechung, Änderung und/oder Ablenkung eines von einer an der Pumpe angeordneten Lichtquelle emittierten Lichtstrahls durch zumindest einen der Klemmabschnitte anspricht.

Wird als Sensor ein Lichtsensor bzw. ein optischer Sensor eingesetzt, ist es in vorteilhafter Weise möglich, die Anzahl der zu messenden Zustände der Schlauchklemme zu vergrößern, indem die Einteilung des durch den Sensor messbaren Wertebereichs verfeinert wird. Wird beispielsweise ein messbarer Wellenlängenbereich oder Beleuchtungsstärkenbereich in mehr als zwei Teilen eingeteilt, können nicht nur die geschlossene und die geöffnete Relativposition der in der Aufnahme aufgenommenen Klemmabschnitte erfasst werden, sondern können auch weitere Zwischenpositionen erfasst werden.

Wie oben beschrieben, kann als Sensor auch ein Tastsensor verwendet werden. Der Tastsensor spricht insbesondere auf den Kontakt mit dem ersten Klemmabschnitt bzw. mit dem Vorsprung am ersten Klemmabschnitt an.

Wird der Sensor als Tastsensor ausgebildet, kann das an der Schlauchklemme vorgesehene Gegenstück zum Sensor, welches vom Sensor erfasst wird, robust ausgebildet werden, ohne besondere optische oder anderweitige Materialeigenschaften zu haben.

Eine erfindungsgemäße Schlauchklemme weist zwei relativ zueinander bewegliche Klemmabschnitte auf, die im Bezug aufeinander in eine geschlossene Relativposition bringbar sind, in welcher der zwischen den Klemmabschnitten angeordnete Schlauch so abgeklemmt ist, dass durch das Innere des Schlauchs kein Medium strömen kann, und die im Bezug aufeinander in eine geöffnete Relativposition bringbar sind, in welcher der zwischen den Klemmabschnitten angeordnete Schlauch nicht abgeklemmt ist, so dass durch das Innere des Schlauchs Medium strömen kann. Zumindest einer der Klemmabschnitte ist mit einer erfindungsgemäßen medizinischen Pumpe verbindbar. Ein erster der zwei Klemmabschnitte weist einen Abschnitt auf, der eine an dem zweiten der Klemmabschnitte vorgesehene Blende bei geschlossener Relativposition der Klemmabschnitte verdeckt und bei geöffneter Relativposition der Klemmabschnitte zumindest teilweise frei gibt oder nicht verdeckt.

Die Blende ist insbesondere eine in dem zweiten Klemmabschnitt vorgesehene Öffnung oder Aussparung, durch welche ein Lichtstrahl bei geöffneter Relativposition der Klemmabschnitte der in einer Aufnahme einer erfindungsgemäßen medizinischen Pumpe aufgenommenen Schlauchklemme in einer ersten Richtung hindurch strahlen kann und welche bei geschlossener Relativposition der Klemmabschnitte der in der Aufnahme aufgenommenen Schlauchklemme derart von dem ersten Klemmabschnitt verdeckt ist, dass zumindest in der ersten Richtung ein Lichtstrahl nicht mehr oder nur noch teilweise durch die Blende hindurch strahlen kann.

Der Vorteil der mit einer Blende ausgestatteten Schlauchklemme ist, dass deren Öffnungszustand mittels eines Lichtsensors berührungslos feststellbar ist.

Alternativ weist eine erfindungsgemäße Schlauchklemme zwei relativ zueinander bewegliche Klemmabschnitte auf, von denen zumindest einer so ausgebildet ist, dass er mit einer anmeldungsgemäßen medizinischen Pumpe werkzeuglos verbunden und insbesondere werkzeuglos von einer anmeldungsgemäßen medizinischen Pumpe abmontiert werden kann. An der Schlauchklemme ist ein Rastelement vorgesehen, mittels dessen die Klemmabschnitte der Schlauchklemme in einer geschlossenen Relativposition gehalten werden können bzw. die Schlauchklemme in einer geschlossenen Stellung gehalten werden kann. In ihrer geschlossenen Relativposition können die Klemmabschnitte einen zwischen sich angeordneten, zumindest abschnittsweise, flexiblen Schlauch so abklemmen, dass durch das Innere des Schlauchs kein Medium fließen kann.

Zusätzlich zu dem Rastelement ist an der Schlauchklemme, an einem ersten der beiden Klemmabschnitte, ein Vorsprung, vorgesehen, der in der geschlossenen Relativposition derart in Richtung des zweiten der beiden Klemmabschnitte ragt, dass er sich bis zu einer vom ersten Klemmabschnitt abgewandten Rückseite des zweiten Klemmabschnitts hin erstreckt. Ist die erfindungsgemäße Schlauchklemme mit der Pumpe verbunden, kann die geschlossene Relativposition der Klemmabschnitte mittels eines an der Rückseite des zweiten Klemmabschnitts an der medizinischen Pumpe angeordneten Sensors detektiert werden.

Das Rastelement kann Teile aufweisen, von denen ein Teil an dem ersten Klemmabschnitt und von denen der andere Teil am zweiten Klemmabschnitt vorgesehen ist. In der geschlossenen Relativposition der Klemmabschnitte wirken die beiden Teile des Rastelements, insbesondere formschlüssig, zusammen und verhindern somit eine Relativbewegung der beiden Klemmabschnitte.

Wird die erfindungsgemäße Schlauchklemme an einen Schlauch angebracht, welcher an einer medizinischen Pumpe angeschlossen ist, ermöglicht die Bauweise der erfindungsgemäßen Schlauchklemme eine einfache Überwachung des Zustands der Schlauchklemme. Wird die Schlauchklemme derart ausgebildet, dass der zweite Klemmabschnitt der mit der Aufnahme der Pumpe verbindbare Klemmabschnitt ist, kann der Sensor in bzw. an der Aufnahme der Pumpe angeordnet werden, wodurch ein einfaches Anbringen der Schlauchklemme in der Aufnahme ermöglicht werden kann.

Die Schlauchklemme kann so ausgebildet sein, dass sich der Vorsprung in der geschlossenen Relativposition durch eine durchgängige Aussparung bzw. ein Loch im zweiten Klemmabschnitt hindurch erstreckt.

Indem eine durchgängige Aussparung im zweiten Klemmabschnitt vorgesehen wird, in welche der Vorsprung des ersten Klemmabschnitts bei der geschlossenen Relativposition eintaucht, kann bei der Herstellung der Schlauchklemme verursachter Verzug leichter und zuverlässiger detektiert werden.

Die Klemmabschnitte der erfindungsgemäßen Schlauchklemme können miteinander mittels eines Scharniers schwenkbar verbunden sein. Wie oben beschrieben, kann das Rastelement aus zwei Teilen bestehen, von denen einer der Teile an dem ersten Klemmabschnitt und der zweite Teil an dem zweiten Klemmabschnitt vorgesehen ist. Die Teile des Rastelements können jeweils integral mit dem entsprechenden Klemmabschnitt ausgebildet sein. Die Teile des Rastelements können jeweils an der vom Scharnier abgewandten Seite eines jeden der beiden Klemmabschnitte angeordnet sein. Zwischen der dem Rastelement zugewandten und der dem Scharnier zugewandten Seite eines jeden Klemmabschnitts kann eine, insbesondere rinnenförmige oder nutförmige, Schlauchaufnahme vorgesehen sein, an deren Fläche der abzuklemmenden Schlauch zumindest abschnittsweise anliegen kann. Sind die Aussparung und der Vorsprung jeweils zwischen dem Scharnier und der jeweiligen Schlauchaufnahme des entsprechenden Klemmelements angeordnet, kann mittels des Vorsprungs und mittels des Rastmittels die Unversehrtheit des Scharniers überwacht werden.

Wird der Schlauch in die Schlauchaufnahmen der Schlauchklemme eingelegt und werden die Klemmabschnitte anschließend soweit aufeinander zu bewegt, dass die Teile des Rastelements ineinander einrasten, kann ein Anwender das Einrasten durch das resultierende Einrastgeräusch wahrnehmen. Wird nun mittels des Sensors der medizinischen Pumpe festgestellt, dass der Vorsprung nicht so weit in die Aussparung eintaucht, wie es in der geschlossenen Relativposition der Klemmabschnitte zu erwarten ist, kann ein Herstellungsfehler des Scharniers oder kann ein Schaden am Scharnier festgestellt werden.

Ein erfindungsgemäßes, medizinisches System besteht aus einer medizinischen Pumpe nach zumindest einem der oben beschriebenen Aspekte und einer Schlauchklemme nach zumindest einem der oben beschriebenen Aspekte.

Vorteil des erfindungsgemäßen Systems ist, dass nach dosiertem Verabreichen eines Mediums mittels der Pumpe an einen Patienten und nach Abschalten der Pumpe ohne großen Aufwand sichergestellt werden kann, dass die Schlauchklemme geschlossen ist und dass keine Free-Flow-Situation vorliegt.

### Kurzbeschreibung der Zeichnungen

Die vorliegende Erfindung wird im Folgenden anhand bevorzugter Ausführungsbeispiele unter Bezugnahme auf die beigefügten Zeichnungen näher beschrieben. Es zeigen:
Figur 1 eine perspektivische Ansicht einer Schlauchklemme gemäß einer ersten Ausführungsform in einer geöffneten Stellung;
Figur 2 eine Ansicht der in Figur 1 angedeuteten Schnittebene II durch die geöffnete Schlauchklemme in einem an einer medizinischen Pumpe angebrachten Zustand;
Figur 3 eine perspektivische Ansicht der in Figur 1 gezeigten Schlauchklemme in einer geschlossenen Stellung;
Figur 4 eine Ansicht der in Figur 3 angedeuteten Schnittebene IV durch die geschlossene Schlauchklemme in einem an der medizinischen Pumpe angebrachten Zustand;
Figur 5 ein Ablaufdiagramm eines Pumpenklappenöffnungsverfahrens;
Figur 6 eine perspektivische Ansicht einer Schlauchklemme gemäß einer zweiten Ausführungsform in einer geöffneten Stellung;
Figur 7A eine Draufsicht der in Figur 6 gezeigten Schlauchklemme;
Figur 7B eine Seitenansicht der in Figur 6 gezeigten Schlauchklemme;
Figur 8 eine Ansicht der in Figur 6 gezeigten geöffneten Schlauchklemme in einem an einer medizinischen Pumpe angebrachten Zustand;
Figur 9 eine perspektivische Ansicht der in Figur 6 gezeigten Schlauchklemme in einer geöffneten Stellung;
Figur 10A eine Draufsicht der in Figur 9 gezeigten Schlauchklemme;
Figur 10B eine Seitenansicht der in Figur 9 gezeigten Schlauchklemme; und
Figur 11 eine Ansicht der in Figur 9 gezeigten geschlossenen Schlauchklemme in einem an einer medizinischen Pumpe angebrachten Zustand.

### Detaillierte Beschreibung bevorzugter Ausführungsformen

An einem ersten Ende des ersten Klemmabschnitts 4 ist ein Scharnier 8 vorgesehen, mittels dessen der erste Klemmabschnitt mit einem ersten Ende des zweiten Klemmabschnitts 6 schwenkbar verbunden ist.

Die beiden Klemmabschnitte 4 und 6 können aus einem thermoplastischen Kunststoff hergestellt sein und kann das Scharnier 8 als Filmscharnier einteilig mit den beiden Klemmabschnitten 4 und 6 ausgebildet sein.

Alternativ könnte das Scharnier 8 mittels an den ersten Enden der Klemmabschnitte 4 und 6 vorgesehener, zinnenförmig ineinander greifender Vorsprünge oder mittels eines separat gefertigten, anmontierten Scharniers umgesetzt sein.

Der erste Klemmabschnitt 4 weist an einem, seinem ersten Ende gegenüberliegenden, zweiten Ende eine erste Federklemme 10 auf. Der zweite Klemmabschnitt 6 weist an einem, seinem ersten Ende gegenüberliegenden, zweiten Ende eine erste Federklemmenaufnahme 12 auf. Die erste Federklemme 10 und die erste Federklemmenaufnahme 12 bilden zusammen ein Rastelement 14.

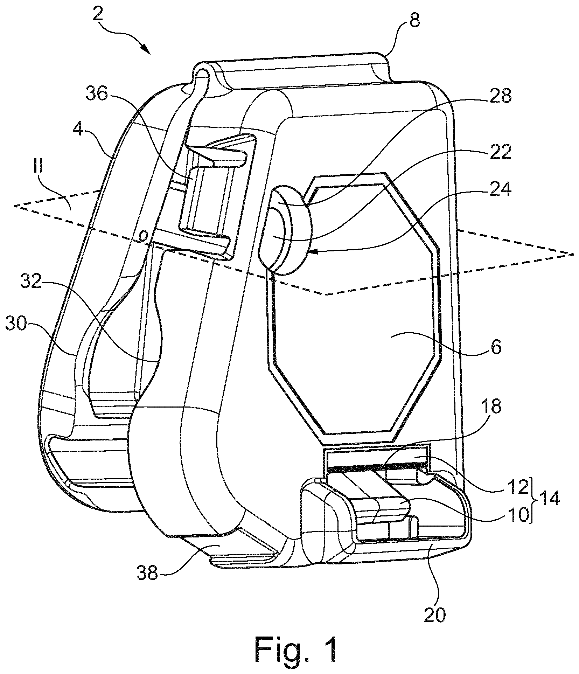

Die erste Federklemme 10 erstreckt sich vom zweiten Ende des ersten Klemmabschnitts 4 aus im Wesentlichen senkrecht zum ersten Klemmabschnitt 4 in Richtung des zweiten Klemmabschnitts 6 und weist ein rampenförmiges freies Ende auf. Werden die beiden Klemmabschnitte 4 und 6 von einer in Figur 1 gezeigten, offenen Stellung aus aufeinander zu bewegt, wirken die beiden zweiten Enden der Klemmabschnitte 4 und 6 ab einem, zwischen den Klemmabschnitten 4 und 6 eingeschlossenen ersten Schließwinkel so zusammen, dass die erste Federklemme 10 mittels ihres rampenförmigen freien Endes durch die erste Federklemmenaufnahme 12 in eine erste Schwenkrichtung verschwenkt wird. Ab einem, kleineren, zweiten Schließwinkel, schwenkt die erste Federklemme 10 in eine, der ersten Schwenkrichtung entgegen gesetzte, zweite Schwenkrichtung zurück und bewirkt mittels einer hinter dem rampenförmigen freien Ende angeordneten Hinterschneidung 16 einen Formschluss mit der ersten Federklemmenaufnahme 12.

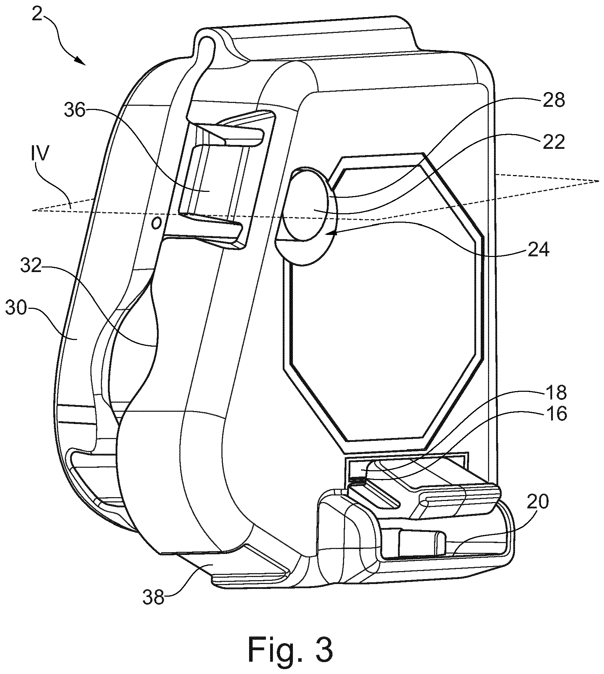

Sind die beiden zweiten Enden der Klemmabschnitte 4 und 6 miteinander über Formschluss verbunden, befindet sich die Schlauchklemme 2 in einer in Figur 3 gezeigten, geschlossenen Stellung bzw. befinden sich die beiden Klemmabschnitte 4 und 6 in einer geschlossenen Relativposition.

Um den Formschluss zwischen den beiden zweiten Enden der beiden Klemmabschnitte 4 und 6 zu lösen, muss die erste Federklemme 10 in die erste Schenkrichtung bewegt werden, so dass die Hinterschneidung 16 an der ersten Federklemme 10 außer Eingriff mit der ersten Federklemmenaufnahme 12 kommt.

Die erste Federklemmenaufnahme 12 ist mittels einer sich im Wesentlichen senkrecht zum zweiten Klemmabschnitt erstreckenden Durchgangsöffnung am zweiten Ende des zweiten Klemmabschnitts 6 umgesetzt, welche eine Kante 18 aufweist, die mit der Hinterschneidung 16 formschlüssig zusammenwirken kann. An der vom ersten Klemmabschnitt 4 abgewandten Seite der Durchgangsöffnung ist am Rand der Durchgangsöffnung eine Umfassung 20 vorgesehen, welche in der geschlossenen Stellung der Schlauchklemme 2 das freie Ende der ersten Federklemme 12 von drei Seiten abschirmt und einen seitlichen Zugriff auf die erste Federklemme 12 im Wesentlichen nur in Richtung der ersten Schwenkrichtung zulässt.

Das Scharnier 8 ist derart ausgebildet, dass es in der in Figur 3 gezeigten geschlossenen Stellung gespannt und in der in der Figur 1 gezeigten offenen Stellung nicht gespannt ist. Alternativ wäre es natürlich auch möglich, dass das Scharnier 8 in der geschlossenen Stellung der Schlauchklemme 2 nicht gespannt ist.

An einem dem ersten Ende des ersten Klemmabschnitts 4 zugewandeten Teil des ersten Klemmabschnitts 4 ist ein sich im Wesentlichen senkrecht zum ersten Klemmabschnitt 4 in Richtung des zweiten Klemmabschnitts 6 erstreckender Taststift 22 vorgesehen.

An einem dem ersten Ende des zweiten Klemmabschnitts 6 zugewandten Teil des zweiten Klemmabschnitts 6 ist eine sich im Wesentlichen senkrecht zum zweiten Klemmabschnitt 6 erstreckende Taststiftaufnahme 24 vorgesehen.

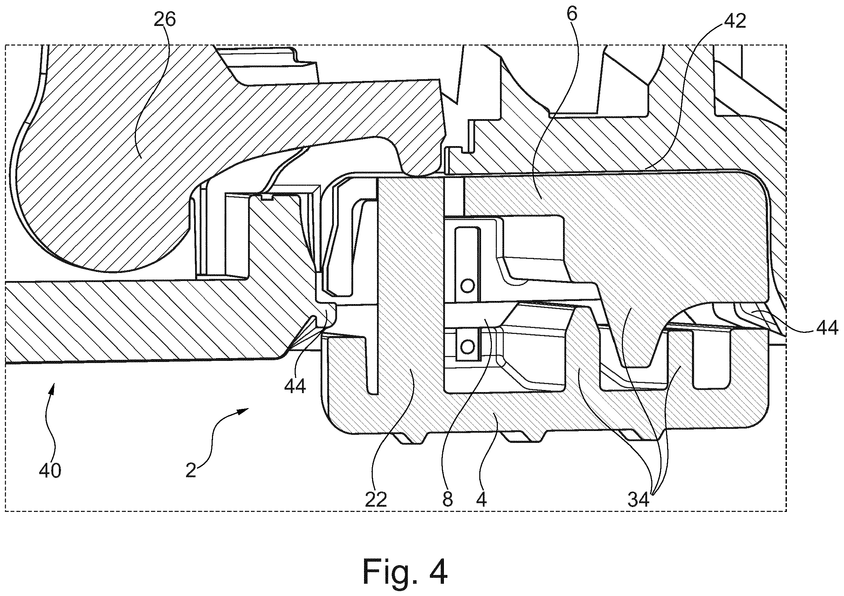

Der kreiszylindrische Taststift 22 und die durchgangsöffnungsförmige Taststiftaufnahme 24 sind derart dimensioniert, dass sich das freie Ende des Taststifts 22 in der geschlossenen Stellung der Schlauchklemme 2 an der vom ersten Klemmabschnitt 4 abgewandten Seite der Taststiftaufnahme 24 befindet und die Position des freien Endes des Taststifts 22, wie in Figur 4 gezeigt, mittels eines Tastsensors 26 erfasst werden kann, welcher an der vom ersten Klemmabschnitt 4 abgewandten Seite des zweiten Klemmabschnitts 6 angeordnet ist.

Genauer gesagt ist der Taststift 22 ein Vorsprung in Form eines Kreiszylinders, dessen freies Ende sich in der geschlossenen Stellung der Schlauchklemme 2 innerhalb der Taststiftaufnahme 24 in Form einer größtenteils kreisrunden Durchgangsbohrung befindet, wobei eine vom ersten Klemmabschnitt 4 abgewandte Fläche des freien Endes zumindest abschnittsweise koplanar mit der Außenfläche der vom ersten Klemmabschnitt 4 abgewandten Seite des zweiten Klemmabschnitts 6 verläuft (siehe Figuren 3 und 4). In der geschlossenen Stellung ist das freie Ende des Taststifts 22 relativ zur Taststiftaufnahme 24 vorzugsweise exzentrisch angeordnet (d.h. ein Spalt 28 zwischen der Außenfläche des freien Endes des Taststifts 22 und der Innenfläche der Taststiftaufnahme 24 ist auf der dem Scharnier 8 zugewandten Seite der Taststiftaufnahme 24 kleiner als auf der dem Rastelement 14 zugewandten Seite).

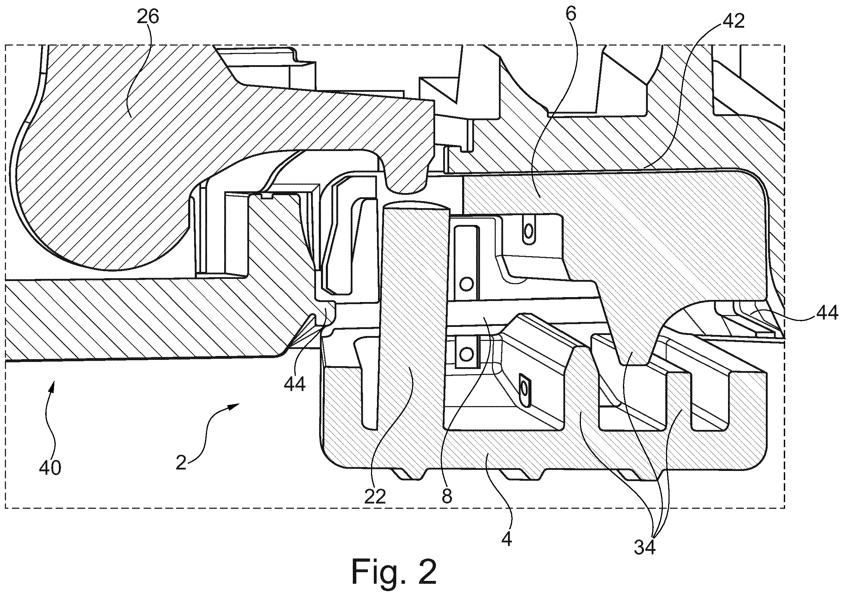

Befindet sich die Schlauchklemme in der in den Figuren 1 und 2 gezeigten offenen Stellung, ist das freie Ende des Taststifts 22 von der Außenfläche der vom ersten Klemmabschnitt 4 abgewandten Seite des zweiten Klemmabschnitts 6 in axialer Richtung des Taststifts 22 bzw. der Taststiftaufnahme 24 beabstandet und ist der Spalt 28 auf der dem Scharnier 8 zugewandten Seite der Taststiftaufnahme 24 größer als auf der dem Rastelement 14 zugewandten Seite.

Zwischen dem Taststift 22 und der ersten Federklemme 10 ist am ersten Klemmabschnitt 4 eine erste, vorzugsweise rinnenförmige, Schlauchaufnahme 30 vorgesehen.

Zwischen der Taststiftaufnahme 24 und der ersten Federklemmenaufnahme 12 ist am zweiten Klemmabschnitt 6 eine zweite, vorzugsweise rinnenförmige, Schlauchaufnahme 32 vorgesehen.

Die beiden Schlauchaufnahmen 30 und 32 sind so ausgebildet, dass sie zumindest abschnittsweise an einem zwischen den Klemmabschnitten 4 und 6 angeordneten Schlauch anliegen, wenn die Klemmabschnitte 4 und 6 in die geschlossene Relativposition gebracht werden.

Wie in den Figuren 2 und 4 gezeigt, sind an den Klemmabschnitten 4 und 6 senkrecht zur Scharnierachse und quer zu den Schlauchaufnahmen 30 und 32 verlaufende Klemmrippen 34 vorgesehen, mittels derer in der geschlossenen Stellung der Schlauchklemme 2 auf den in den Schlauchaufnahmen 30 und 32 aufgenommenen, zumindest in diesem Bereich flexiblen Schlauch lokal Druck ausgeübt wird, so dass der Schlauch zusammengedrückt und das Innere des Schlauch im Bereich der Klemmrippen 34 gesperrt ist. Die Klemmrippen 34 können so angeordnet sein, dass sie sich nicht nur in Richtung senkrecht zur Scharnierachse, sondern auch in Richtung parallel zur Scharnierachse zwischen dem Taststift 22 bzw. der Taststiftaufnahme 24 und dem Rastelement 14 erstrecken.

An den seitlichen Rändern des zweiten Klemmabschnitts 6 ist zumindest eine zweite Federklemme 36 und zumindest eine zweite Federklemmenaufnahme 38 vorgesehen.

Ist die Schlauchklemme 2 an einer medizinischen Pumpe 40 angebracht, wird der zweite Klemmabschnitt 6 in einer pumpenseitigen Klemmabschnittaufnahme 42 aufgenommen, deren Grundriss dem Grundriss des zweiten Klemmabschnitts 6 entspricht. Die Verbindung zwischen der Schlauchklemme 2 und der Pumpe 40 wird durch formschlüssiges Zusammenwirken von Vorsprüngen 44 bzw. Federklemmen am Rand der Klemmabschnittaufnahme 42 mit der zweiten Federklemme 36 und/oder der zweiten Federklemmaufnahme 38 gewährleistet.

Wie in Figur 2 gezeigt, ist der Tastsensor 26 derart ausgebildet, dass ein Taster 46 des Tastsensors 26 von der dem ersten Klemmabschnitt 4 abgewandten Seite her in die Taststiftaufnahme 24 der geöffneten, an der Pumpe 40 angebrachten Schlauchklemme 2 hineinragt. Wird die an der Pumpe 40 angebrachte Schlauchklemme 2 geschlossenen, wird der Taster 46 vom Taststift 22 des ersten Klemmabschnitts 2 aus der Taststiftaufnahme 24 verdrängt (siehe Figur 4). Je nachdem, wie weit der Taster 46 aus der Taststiftaufnahme 24 bewegt wird, gibt der Tastsensor 24 ein dem Öffnungsgrad der beiden Klemmabschnitte 4 und 6 entsprechendes Signal (0 oder 1) an eine (nicht gezeigte) elektronische Steuereinheit in der Pumpe 40 weiter.

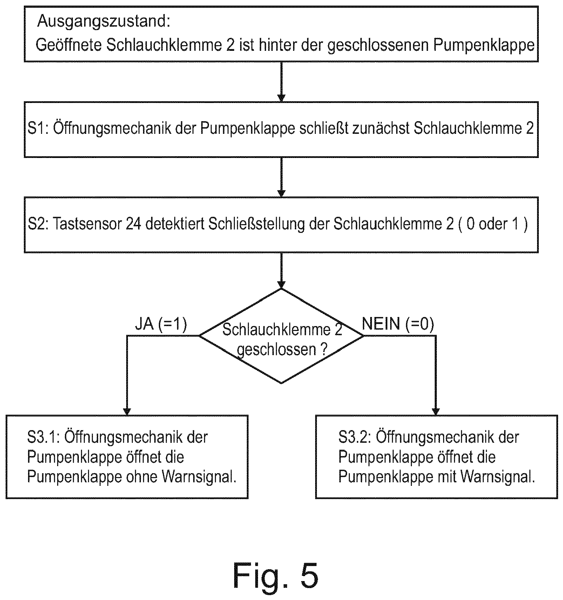

In Figur 5 ist ein beispielhaftes Pumpenklappenöffnungsverfahren veranschaulicht. Im betriebsbereiten Zustand der Pumpe 40, in welchem ein Schlauch an der Pumpe 40 angeschlossen und mittels der Schlauchklemme 2 gesichert ist, befindet sich die geöffnete Schlauchklemme hinter der geschlossenen Pumpenklappe (siehe Ausgangszustand in Figur 5).

Wird eine Öffnungsmechanik der Pumpenklappe betätigt (siehe Schritt S1 in der Figur 5), bewirkt die Öffnungsmechanik der Pumpenklappe zunächst das Schließen der Schlauchklemme 2, indem sie auf das Rastelement 14 der Schlauchklemme 2 einwirkt.

Wird mittels des Tastsensors 26 detektiert, dass die Schlauchklemme 2 ordnungsgemäß geschlossen ist (siehe Schritt S2 in der Figur 5), wird beim eigentlichen Öffnen der Pumpenklappe mittels der Öffnungsmechanik kein Warnsignal ausgegeben (siehe Schritt S3.1 in der Figur 5).

Wird mittels des Tastsensors 26 detektiert, dass die Schlauchklemme 2 nicht ordnungsgemäß geschlossen ist (siehe Schritt S2 in der Figur 5), wird beim eigentlichen Öffnen der Pumpenklappe mittels der Öffnungsmechanik ein Warnsignal ausgegeben (siehe Schritt S3.2 in der Figur 5).

Dieses Warnsignal kann mithilfe einer optischen Anzeige (z.B. Blinklicht) und/oder eines Lautsprechers erfolgen.

Die Öffnungsmechanik der Pumpenklappe kann so ausgebildet sein, dass ein Öffnen der Pumpenklappe auch ein Abschalten der Pumpe bewirkt.

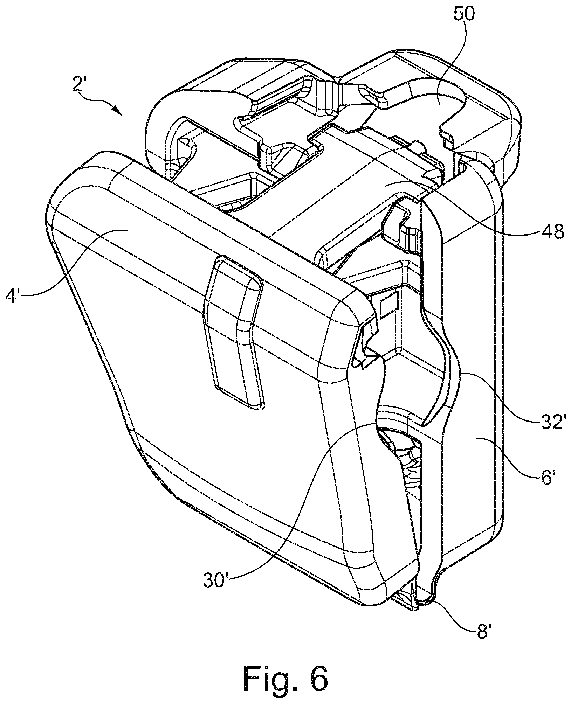

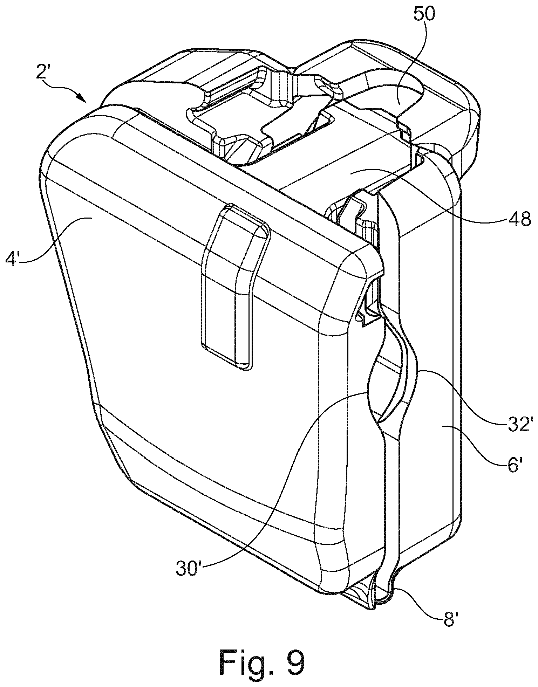

Eine Schlauchklemme 2' gemäß einer zweiten Ausführungsform der Erfindung ist in den Figuren 6 bis 11 gezeigt. Die Schlauchklemme 2' weist ebenfalls zwei miteinander über ein Filmscharnier 8' verbundene Klemmabschnitte 4' und 6' auf.

An dem vom Filmscharnier 8' abgewandten Ende des ersten Klemmabschnitts 4' ist ein Verdeckabschnitt 48 vorgesehen, welcher sich in der geschlossenen Relativposition der beiden Klemmabschnitte 4' und 6' in Richtung des zweiten Klemmabschnitts 6' erstreckt.

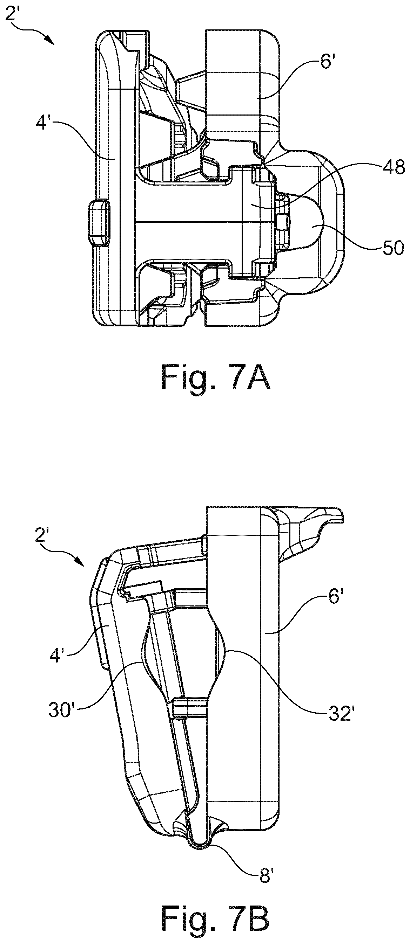

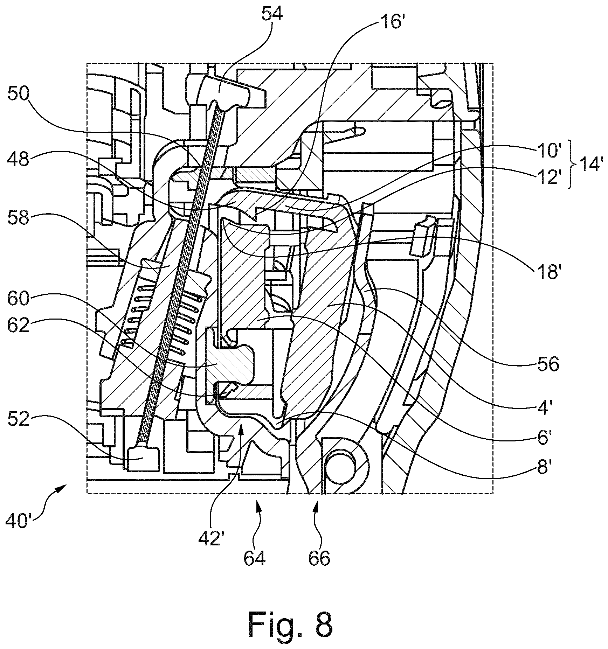

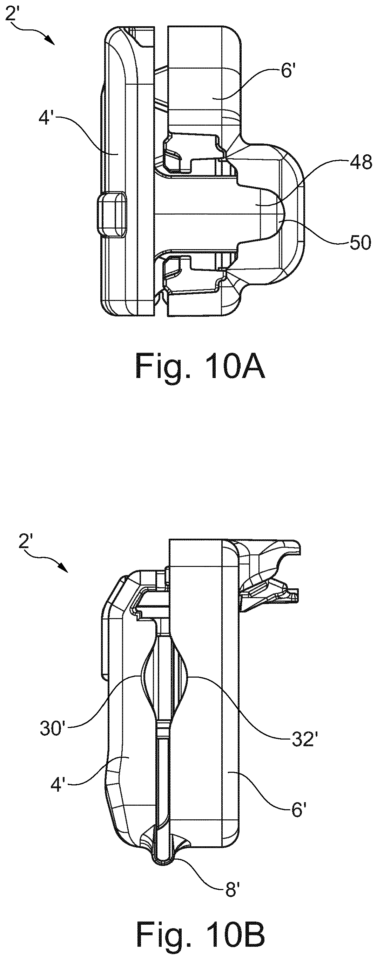

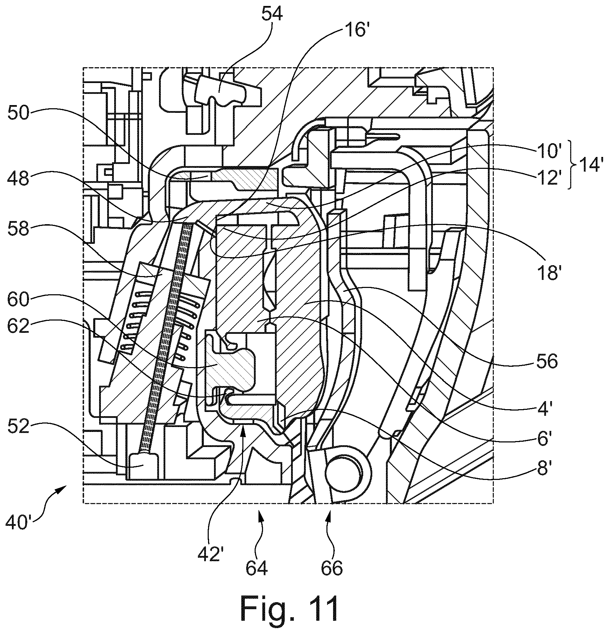

An dem vom Filmscharnier 8' abgewandten Ende des zweiten Klemmabschnitts 6' ist eine Blende 50 vorgesehen, welche bei geschlossener Relativposition der beiden Klemmabschnitte 4' und 6' von dem Verdeckabschnitt verdeckt wird. Verdeckt heißt in diesem Zusammenhang, dass ein parallel zur Haupterstreckungsrichtung des zweiten Klemmabschnitts 6' verlaufender Lichtstrahl, der bei geöffneter Schlauchklemme 2' durch die Blende 50 hindurchtritt (siehe Figuren 7A und 8), bei geschlossener Schlauchklemme 2' auf den Verdeckabschnitt 50 trifft (siehe Figuren 10A und 11).

Wie die Schlauchklemme 2 gemäß der ersten Ausführungsform weist auch die Schlauchklemme 2' gemäß der zweiten Ausführungsform an ihrem ersten Klemmabschnitt 4' zwischen seinem dem Filmscharnier 8' zugewandten Ende und seinem dem Filmscharnier 8' abgewandten Ende eine erste Schlauchaufnahme 30' in Form einer rinnenförmigen Vertiefung auf (siehe Figuren 6, 7B, 9 und 10B) und an ihrem zweiten Klemmabschnitt 6' zwischen seinem dem Filmscharnier 8' zugewandten Ende und seinem dem Filmscharnier 8' abgewandten Ende eine zweite Schlauchaufnahme 32' in Form einer rinnenförmigen Vertiefung auf (siehe Figuren 6, 7B, 9 und 10B).

Wie in den Figuren 8 und 11 gezeigt, wird der Verdeckabschnitt 48 durch eine erste Federklemme 10' gebildet, welche zusammen mit einer ersten Federklemmenaufnahme 12' ein Rastelement 14' bildet. Das Rastelement 14' gemäß der zweiten Ausführungsform entspricht hinsichtlich seiner Rastfunktion dem Rastelement 14 gemäß der ersten Ausführungsform. So ist an der ersten Federklemme 10' eine Hinterschneidung 16' vorgesehen, welche bei geschlossener Schlauchklemme 2' mit einer Kante 18' an der ersten Federklemmenaufnahme 12' formschlüssig zusammenwirkt.

In Figur 8 ist die in einer Schlauchklemmenaufnahme 42' aufgenommene Schlauchklemme 2' in einem geöffneten Zustand gezeigt. Die Schlauchklemmenaufnahme 42' ist an einem Grundkörper 64 einer Pumpe 40' vorgesehen und wird von einer am Grundkörper 64 angebrachten Pumpenklappe 66 abgedeckt, wenn sich die Pumpenklappe 66, wie in den Figuren 8 und 11 gezeigt, in einer geschlossenen Stellung befindet. In der Schlauchklemmenaufnahme 42' ist ein Druckknopf oder Clip 60 in Form eines Hafenpollers vorgesehen, welcher bei einer in der Schlauchklemmenaufnahme 42' aufgenommenen Schlauchklemme 2' mit einer Clipaufnahme 62 im zweiten Klemmabschnitt 6' formschlüssig zusammenwirkt. Die Clipaufnahme 62 weist die Form einer runden Öffnung auf, deren Rand bzw. Federlaschen elastisch erweiterbar ist, wodurch ein mehrfaches Einlegen und Entnehmen ermöglicht wird.

In der Pumpenklappe 66 ist ein erstes Aktorelement 56 in Form eines Schwenkhebels vorgesehen, dessen Schwenkachse im Wesentlichen parallel zur Scharnierachse des Filmscharniers 8' verläuft. Mittels des ersten Aktorelements 56 kann der erste Klemmabschnitt 4' von einer geöffneten Relativposition in Richtung des zweiten Klemmabschnitts 6' in eine geschlossene Relativposition verschwenkt werden.

In dem Grundkörper 64 ist ein zweites Aktorelement 58 in Form eines linear verschiebbaren Riegels vorgesehen, dessen Verschieberichtung quer zur ersten Federklemme 10' verläuft. Mittels des zweiten Aktorelements 58 kann die erste Federklemme 10' bei geschlossener Relativposition der beiden Klemmabschnitte 4' und 6' derart bewegt werden, dass die Hinterschneidung 16' an der ersten Federklemme 10' am ersten Klemmabschnitt 4' außer Eingriff mit der Kante 18' an der ersten Federklemmenaufnahme 12' am zweiten Klemmabschnitt 6' kommen kann. Sind die Hinterschneidung 16' und die Kante 18' außer Eingriff, bewegt sich der erste Klemmabschnitt 4' in die geöffnete Relativposition, sobald in einem von der Schlauchklemme 2' eingeklemmten Schlauch ein bestimmter Mindestdruck vorliegt. Alternativ kann die Schlauchklemme 2' auch derart ausgebildet sein, dass sich die Klemmabschnitte 4' und 6' in einem spannungsfreien Zustand in der geöffneten Relativposition befinden.

In dem Grundkörper 64 ist einerseits des vom Filmscharnier 8' abgewandten Endes des zweiten Klemmabschnitts 6' eine Lichtquelle 52 und andererseits des besagten Endes ein Lichtsensor 54 vorgesehen. Unter dem Begriff ,Licht' wird im Zusammenhang dieser Beschreibung und der Ansprüche jegliche elektromagnetische Strahlung verstanden, welche mittels Kunststoff oder metallisierten Kunststoffs abgeschirmt werden kann. Somit zeichnet sich der Lichtsensor 54 allgemein dadurch aus, dass er zumindest eine Eigenschaftsänderung bzw. eine Eigenschaft der Lichts, wie beispielsweise Intensität, detektieren kann. Die Lichtquelle 52 und der Lichtsensor 54 sind in dem Grundkörper 64 derart angeordnet, dass ein Lichtstrahl von der Lichtquelle 52 durch die Blende 50 hindurch zum Lichtsensor 54 strahlen kann, wenn sich die Klemmabschnitte 4' und 6' in der geöffneten Relativposition befinden, und dass ein Lichtstrahl von der Lichtquelle 52 auf den Verdeckabschnitt 48 trifft und nicht zum Lichtsensor 54 strahlen kann, wenn sich die Klemmabschnitte 4' und 6' in der geschlossenen Relativposition befinden.

Die in den Figuren 1 bis 11 gezeigten und oben beschriebenen Ausführungsformen der erfindungsgemäßen Schlauchklemme und der erfindungsgemäßen medizinischen Pumpe stellen lediglich eine mögliche Umsetzung der beanspruchten Erfindung dar.

Beispielsweise muss der Taststift 22 keine kreiszylindrische Form aufweisen, sondern kann auch allgemein zylindrisch oder rotationskörperförmig sein. Entsprechend kann auch der Querschnitt der Taststiftaufnahme 24 polygonförmig oder oval sein.

Die Schlauchklemme 2 ist einstückig. Alternativ ist es auch möglich, die Schlauchklemme 2 durch Zusammenfügen mehrerer Einzelteile zu fertigen.

Bei der beschriebenen Ausführungsform wird der Öffnungsgrad der Schlauchklemme 2 anhand der Verschiebung des Tasters 46 in axialer Richtung der Taststiftaufnahme 24 erfasst. Alternativ oder zusätzlich wäre es möglich, mittels des Tasters 46 die Verschiebung des Taststifts 22 relativ zur Taststiftaufnahme 24 quer zur axialen Richtung der Taststiftaufnahme 24 zu erfassen (Ermitteln der Breite des Spalts 28).

### Bezugszeichenliste

- 2; 2': Schlauchklemme
- 4; 4': erster Klemmabschnitt
- 6; 6': zweiter Klemmabschnitt
- 8; 8': Scharnier
- 10; 10': erste Federklemme
- 12; 12': erste Federklemmenaufnahme
- 14; 14': Rastelement
- 16; 16': Hinterschneidung
- 18; 18': Kante
- 20: Umfassung
- 22: Taststift
- 24: Taststiftaufnahme
- 26: Tastsensor
- 28: Spalt
- 30; 30': erste Schlauchaufnahme
- 32; 32': zweite Schlauchaufnahme
- 34: Klemmrippe
- 36: zweite Federklemme
- 38: zweite Federklemmenaufnahme
- 40; 40': Pumpe
- 42; 42': Schlauchklemmen- bzw. Klemmabschnittaufnahme
- 44: Vorsprung
- 46: Taster
- 48: Verdeckabschnitt
- 50: Blende
- 52: Lichtquelle
- 54: Lichtsensor
- 56: erstes Aktorelement
- 58: zweites Aktorelement
- 60: Clip
- 62: Clipaufnahme
- 64: Grundkörper
- 66: Pumpenklappe

## Patentansprüche

1. Medizinische Pumpe (40; 40'), zum Fördern eines Mediums, mit einer Aufnahme (42; 42') für eine von der Pumpe (40; 40') separate, an einem zumindest abschnittsweise flexiblen Schlauch angeordnete Schlauchklemme (2; 2'), nach einem der Ansprüche 12 oder 13-15, welche zwei relativ zueinander bewegliche Klemmabschnitte (4, 6; 4', 6') aufweist, die im Bezug aufeinander in eine geschlossene Relativposition bringbar sind, in welcher der zwischen den Klemmabschnitten (4, 6; 4', 6') angeordnete Schlauch so abgeklemmt ist, dass durch das Innere des Schlauchs kein Medium strömen kann, und die im Bezug aufeinander in eine geöffnete Relativposition bringbar sind, in welcher der zwischen den Klemmabschnitten (4, 6; 4', 6') angeordnete Schlauch nicht abgeklemmt ist, so dass durch das Innere des Schlauchs Medium strömen kann; wobei die medizinische Pumpe (40, 40') einen, insbesondere in der Aufnahme (42; 42') vorgesehenen, Sensor (26; 54), welcher bei der in der Aufnahme (42; 42') aufgenommenen Schlauchklemme (2; 2') zumindest erfasst, ob sich deren Klemmabschnitte (4, 6; 4', 6') in der geschlossenen oder der geöffneten Relativposition befinden, und eine elektronische Datenverarbeitungseinheit aufweist,
**dadurch gekennzeichnet, dass**
die elektronische Datenverarbeitungseinheit die zum Einsatz der Schlauchklemme (2; 2') erforderliche strukturelle und/oder funktionelle Integrität der in der Aufnahme (42; 42') aufgenommenen Schlauchklemme (2; 2') anhand des zeitlichen Verlaufs der durch den Sensor (26; 54) beim Übergang der Klemmabschnitte (4, 6; 4', 6') von der geschlossenen zur geöffneten oder von der geöffneten zur geschlossenen Relativposition durchgeführten Messung erfasst, und mit einem zeitlichen Soll-Verlauf der Messung vergleicht.

2. Medizinische Pumpe (40; 40') nach Anspruch 1,
**gekennzeichnet durch**
einen Aktor (58), welcher die Klemmabschnitte (4, 6; 4'; 6') der in der Aufnahme (42; 42') aufgenommenen Schlauchklemme (2; 2') von der geschlossenen in die geöffnete und/oder von der geöffneten in die geschlossene Relativposition bringen kann.

3. Medizinische Pumpe (40; 40') nach Anspruch 1 oder 2,
**gekennzeichnet durch**
eine Pumpenklappe (66), welche in einer geschlossenen Stellung die Aufnahme (42; 42') derart abdeckt, dass die Schlauchklemme (2; 2') nicht aus der Aufnahme (42; 42') entnehmbar oder nicht in die Aufnahme (42; 42') einbringbar ist, und in einer geöffneten Stellung die Aufnahme (42; 42') derart freigibt, dass die Schlauchklemme (2; 2') aus der Aufnahme (42; 42') entnehmbar oder in die Aufnahme (42; 42') einbringbar ist.

4. Medizinische Pumpe (40; 40') nach Ansprüchen 2 und 3,
**dadurch gekennzeichnet, dass**
der Aktor (56, 58) die Klemmabschnitte (4, 6; 4', 6') der in der Aufnahme (42; 42') aufgenommenen Schlauchklemme (2; 2') bei geschlossener Stellung der Pumpenklappe (66) von der geschlossenen in die geöffnete und/oder von der geöffneten in die geschlossene Relativposition bringen kann.

5. Medizinische Pumpe (40; 40') nach einem der Ansprüche 2 bis 4,
**dadurch gekennzeichnet, dass**
die geöffnete Relativposition der Klemmabschnitte (4, 6; 4', 6') der in der Aufnahme (42; 42') aufgenommenen Schlauchklemme (2; 2') durch den Aktor (56, 58) derart veränderlich ist, dass ein Öffnungsgrad des Inneren des Schlauchs und somit ein Volumenstrom des durch den Schlauch strömenden Mediums einstellbar ist.

6. Medizinische Pumpe (40; 40') nach Anspruch 5,
**dadurch gekennzeichnet, dass**
der Sensor (26; 54) die Änderung der geöffneten Relativposition der Klemmabschnitte (4, 6; 4', 6') der in der Aufnahme (42; 42') aufgenommenen Schlauchklemme (2; 2') detektieren kann, so dass mittels des Sensors (26; 54) der Öffnungsgrad des Inneren des Schlauchs und somit der Volumenstrom des durch den Schlauch strömenden Mediums erfassbar ist.

7. Medizinische Pumpe (40; 40') nach einem der Ansprüche 1 bis 6,
**dadurch gekennzeichnet, dass**
der Sensor (26; 54) die geschlossene Relativposition der beiden Klemmabschnitte (4, 6; 4', 6') erfasst, indem er die Position eines ersten (4; 4') der beiden Klemmabschnitte (4, 6; 4', 6') detektiert, und
die Aufnahme (42; 42') derart ausgestaltet ist, dass in ihr der zweite (6; 6') der beiden Klemmabschnitte (4, 6; 4', 6') fixiert werden kann.

8. Medizinische Pumpe (40; 40') nach den Ansprüchen 3 und 7,
**dadurch gekennzeichnet, dass**
die Pumpe (40; 40') einen Grundkörper (64) aufweist, demgegenüber die Pumpenklappe (66) relativ beweglich ist,
die Aufnahme (42; 42') an dem Grundkörpers (64) vorgesehen ist, und
der erste Klemmabschnitt (4; 4') mittels der Pumpenklappe (66) bewegbar ist.

9. Medizinische Pumpe (40') nach einem der Ansprüche 1 bis 8,
**dadurch gekennzeichnet, dass**
der Sensor (54) ein Lichtsensor (54) ist, der auf eine Unterbrechung, Änderung und/oder Ablenkung eines von einer an der Pumpe (40') angeordneten Lichtquelle (52) emittierten Lichtstrahls durch zumindest einen der Klemmabschnitte (4', 6') anspricht.

10. Medizinische Pumpe (40) nach einem der Ansprüche 1 bis 8,
**dadurch gekennzeichnet, dass**
der Sensor (26) ein Tastsensor (26) ist, der auf den Kontakt des Sensors (26) mit zumindest einem der Klemmabschnitte (4, 6) anspricht.

11. Medizinische Pumpe (40) nach einem der Ansprüche 1 bis 10,
**dadurch gekennzeichnet, dass**
in der Aufnahme (42') ein Druckknopf oder Clip (60) vorgesehen ist, welcher bei der in der Aufnahme (42') aufgenommenen Schlauchklemme (2') mit einer in einem der Klemmabschnitte (6') ausgebildeten Clipaufnahme (62) zur lösbaren Fixierung formschlüssig zusammenwirkt.

12. Schlauchklemme (2') mit zwei relativ zueinander beweglichen Klemmabschnitten (4', 6'), die im Bezug aufeinander in eine geschlossene Relativposition bringbar sind, in welcher der zwischen den Klemmabschnitten (4', 6') angeordnete Schlauch so abgeklemmt ist, dass durch das Innere des Schlauchs kein Medium strömen kann, die im Bezug aufeinander in eine geöffnete Relativposition bringbar sind, in welcher der zwischen den Klemmabschnitten (4', 6') angeordnete Schlauch nicht abgeklemmt ist, so dass durch das Innere des Schlauchs Medium strömen kann, und von denen zumindest ein Klemmabschnitt (6') mit einer medizinischen Pumpe (40; 40') nach einem der Ansprüche 1 bis 11 verbindbar ist,
**dadurch gekennzeichnet, dass**
ein erster (4') der zwei Klemmabschnitte (4', 6') einen Abschnitt aufweist, der eine an dem zweiten (6') der Klemmabschnitte (4', 6') vorgesehene Blende bei geschlossener Relativposition der Klemmabschnitte (4', 6') verdeckt und bei geöffneter Relativposition der Klemmabschnitte (4', 6') zumindest teilweise frei gibt oder nicht verdeckt.

13. Schlauchklemme (2) mit zwei relativ zueinander beweglichen Klemmabschnitten (4, 6), die mittels eines an der Schlauchklemme (2) vorgesehenen Rastelements (14) in einer geschlossenen Relativposition arretierbar sind, in der sie einen zwischen sich angeordneten, flexiblen Schlauch so abklemmen können, dass durch das Innere des Schlauchs kein Medium fließen kann, und von denen zumindest ein Klemmabschnitt (6) mit einer medizinischen Pumpe (40) nach einem der Ansprüche 1 bis 11 verbindbar ist,
**dadurch gekennzeichnet, dass**
zusätzlich zu dem Rastelement (14) an einem ersten (4) der beiden Klemmabschnitte (4, 6) ein Vorsprung (22) vorgesehen ist, der in der geschlossenen Relativposition derart in Richtung des zweiten Klemmabschnitts (6) ragt, dass er sich bis zu einer vom ersten Klemmabschnitt (4) abgewandten Rückseite des zweiten Klemmabschnitts (6) hin erstreckt und die geschlossene Relativposition der Klemmabschnitte (4, 6) der mit der medizinischen Pumpe (40) verbundenen Schlauchklemme (2) mittels eines an der Rückseite des zweiten Klemmabschnitts (6), an der medizinischen Pumpe (40) angeordneten Sensors (26) detektierbar ist.

14. Schlauchklemme (2) nach Anspruch 13,
**dadurch gekennzeichnet, dass**
sich der Vorsprung (22) in der geschlossenen Relativposition durch eine durchgängige Aussparung (24) im zweiten Klemmabschnitt (6) hindurch erstreckt.

15. Schlauchklemme (2) nach Anspruch 13 oder 14,
**dadurch gekennzeichnet, dass**
die Klemmabschnitte (4, 6) miteinander mittels eines Scharniers (8) schwenkbar verbunden sind,
das Rastelement (14) jeweils an der vom Scharnier (8) abgewandten Seite eines jeden der beiden Klemmabschnitte (4, 6) angeordnet ist,
zwischen der dem Rastelement (14) zugewandten und der dem Scharnier (8) zugewandten Seite eines jeden Klemmabschnitts (4, 6) eine Schlauchaufnahme (30, 32) vorgesehen ist, und
die Aussparung (24) und der Vorsprung (22) jeweils zwischen dem Scharnier (8) und der jeweiligen Schlauchaufnahme (30; 32) des entsprechenden Klemmelements (4; 6) angeordnet sind.

16. Medizinisches System bestehend aus einer medizinischen Pumpe (40; 40') nach einem der Ansprüche 1 bis 11 und einer Schlauchklemme (2; 2') nach einem der Ansprüche 12 bis 15.

## Claims

1. A medical pump (40; 40') for conveying a medium, comprising a seating (42; 42') for a hose clamp (2; 2'), according to one of claims 12 or 13 -15, which is separate from the pump (40; 40') and arranged on a hose that is flexible at least in sections, said hose clamp comprising two clamping portions (4, 6; 4', 6') which can be moved relative to each other and can be brought into a closed relative position with respect to each other, in which the hose arranged between the clamping portions (4, 6; 4', 6') is pinched off such that no medium can flow through the interior of the hose, and into an opened relative position with respect to each other, in which the hose arranged between the clamping portions (4, 6; 4', 6') is not pinched off, so that a medium can flow through the interior of the hose,
wherein the medical pump (40; 40') comprises a sensor (26; 54), which is provided in particular in the seating (42; 42') and which, when the hose clamp (2; 2') is provided in the seating (42; 42'), detects at least whether their clamping portions (4, 6; 4', 6') are in the closed or the opened relative position, and comprises an electronic data processing unit,
**characterized in that**
the electronic data processing unit detects the structural and/or functional integrity of the hose clamp (2; 2') received in the seating (42; 42') required for use of the hose clamp (2; 2') with the aid of the temporal course of the measurement carried out by the sensor (26; 54) during the transition of the clamping portions (4, 6; 4', 6') from the closed to the opened or from the opened to the closed relative position, and compares it with a temporal target course of the measurement.

2. The medical pump (40; 40') according to claim 1,
**characterized by**
an actuator (58) which is able to move the clamping portions (4, 6; 4'; 6') of the hose clamp (2; 2') received in the seating (42; 42') from the closed to the opened and/or from the opened to the closed relative position.

3. The medical pump (40; 40') according to claim 1 or 2,
**characterized by**
a pump flap (66) which in a closed position covers the seating (42; 42') such that the hose clamp (2; 2') cannot be removed from the seating (42; 42') or cannot be inserted into the seating (42; 42'), and in an opened position releases the seating (42; 42') such that the hose clamp (2; 2') can be removed from the seating (42; 42') or can be inserted into the seating (42; 42').

4. The medical pump (40; 40') according to claims 2 and 3,
**characterized in that**
the actuator (56, 58), in the closed position of the pump flap (66), can move the clamping portions (4, 6; 4', 6') of the hose clamp (2; 2') which is received in the seating (42; 42') from the closed to the opened and/or from the opened to the closed relative position.

5. The medical pump (40; 40') according to any of claims 2 to 4,
**characterized in that**
the opened relative position of the clamping portions (4, 6; 4', 6') of the hose clamp (2; 2') received in the seating (42; 42') can be altered by the actuator (56, 58) such that a degree of openness of the interior of the hose and hence a volumetric flow of the medium flowing through the hose can be adjusted.

6. The medical pump (40; 40') according to claim 5,
**characterized in that**
the sensor (26; 54) is capable of detecting the alteration of the opened relative position of the clamping portions (4, 6; 4', 6') of the hose clamp (2; 2') received in the seating (42; 42'), so that by means of the sensor (26; 54) the degree of openness of the interior of the hose and hence the volumetric flow of the medium flowing through the hose can be detected.

7. The medical pump (40; 40') according to any of claims 1 to 6,
**characterized in that**
the sensor (26; 54) detects the closed relative position of the two clamping portions (4, 6; 4', 6') by sensing the position of a first one (4; 4') of the two clamping portions (4, 6; 4', 6'), and
the seating (42; 42') is configured such that the second one (6; 6') of the two clamping portions (4, 6; 4', 6') can be fixed therein.

8. The medical pump (40; 40') according to claims 3 and 7,
**characterized in that**
the pump (40; 40') comprises a main body (64) relative to which the pump flap (66) can be moved,
the seating (42; 42') is provided on the main body (64), and
the first clamping portion (4; 4') can be moved by means of the pump flap (66).

9. The medical pump (40') according to any of claims 1 to 8,
**characterized in that**
the sensor (54) is a light sensor (54) which is responsive to at least one of the clamping portions (4', 6') interrupting, changing and/or deflecting a light beam emitted from a light source (52) arranged on the pump (40').

10. The medical pump (40) according to any of claims 1 to 8,
**characterized in that**
the sensor (26) is a tactile sensor (26) which is responsive to the contact of the sensor (26) with at least one of the clamping portions (4, 6).

11. The medical pump (40) according to any of claims 1 to 10,
**characterized in that**
a snap fastener or clip (60) is provided in the seating (42') and for a detachable fixation cooperates in a form-locking manner with a clip seating (62) formed in one of the clamping portions (6') when the hose clamp (2') is received in the seating (42').

12. A hose clamp (2') comprising two clamping portions (4', 6') which can be moved relative to each other to a closed relative position in which the hose arranged between the clamping portions (4', 6') is pinched off such that no medium can flow through the interior of the hose, and into an opened relative position with respect to each other, in which the hose arranged between the clamping portions (4', 6') is not pinched off, so that a medium can flow through the interior of the hose, and from which at least one clamping portion (6') can be connected to a medical pump (40; 40') according to any of the claims 1 to 11,
**characterized in that**
a first one (4') of the two clamping portions (4', 6') comprises a portion which covers an aperture provided on the second one (6') of the clamping portions (4', 6') in a closed relative position of the clamping portions (4', 6') and at least partially releases said aperture or does not cover it in the opened relative position of the clamping portions (4', 6').

13. A hose clamp (2) comprising two clamping portions (4, 6) which can be moved relative to each other and which can be arrested in a closed relative position by means of a latching element (14) provided on the hose clamp (2), in which closed relative position they can pinch off a flexible hose arranged between them such that no medium can flow through the interior of the hose, and from which at least one clamping portion (6) can be connected to a medical pump (40) according to any of the claims 1 to 11,
**characterized in that**
in addition to the latching element (14), a protrusion (22) is provided on a first one (4) of the two clamping portions (4, 6), said protrusion in the closed relative position protruding in such a manner toward the second clamping portion (6) that it extends up to a rear side of the second clamping portion (6) facing away from the first clamping portion (4) and the closed relative position of the clamping portions (4, 6) of the hose clamp (2) connected to the medical pump (40) can be sensed by means of a sensor (26) arranged on the rear side of the second clamping portion (6) on the medical pump (40).

14. The hose clamp (2) according to claim 13,
**characterized in that**
the protrusion (22) extends in the closed relative position through a continuous recess (24) in the second clamping portion (6).

15. The hose clamp (2) according to claim 13 or 14,
**characterized in that**
the clamping portions (4, 6) are pivotally connected to each other by means of a hinge (8),
the latching element (14) is arranged on each of the two clamping portions (4, 6) on the side facing away from the hinge (8),
a hose seating (30, 32) is provided between the side facing the latching element (14) and the side facing the hinge (8) of each clamping portion (4, 6), and
the recess (24) and the protrusion (22) are each arranged between the hinge (8) and the respective hose seating (30; 32) of the corresponding clamping portion (4; 6).

16. A medical system comprising a medical pump (40; 40') according to any of claims 1 to 11 and a hose clamp (2; 2') according to any of claims 12 to 15.

## Revendications

1. Pompe médicale (40 ; 40') pour acheminer un agent, avec un logement (42 ; 42') pour une pince pour tuyaux souples (2 ; 2') séparée de la pompe (40 ; 40') et agencée sur un tuyau souple au moins par sections selon l'une quelconque des revendications 12 ou 13 à 15, qui présente deux sections de pince(4, 6 ; 4', 6') mobiles l'une par rapport à l'autre, qui peuvent être amenées dans une position relative fermée l'une par rapport à l'autre, dans laquelle position le tuyau souple agencé entre les sections de pince (4, 6 ; 4', 6') est pincé de sorte qu'aucun agent ne puisse s'écouler à travers l'intérieur du tuyau souple, et qui peuvent être amenées dans une position relative ouverte l'une par rapport à l'autre, dans laquelle le tuyau souple agencé entre les sections de pince (4, 6 ; 4', 6') n'est pas pincé de sorte qu'un agent puisse s'écouler à travers l'intérieur du tuyau souple ; dans lequel la pompe médicale (40, 40') présente un capteur (26 ; 54) disposé en particulier dans le logement (42 ; 42'), qui détecte au moins dans la pince pour tuyaux souples (2 ; 2') reçue dans le logement (42 ; 42') si ses sections de pince (4, 6 ; 4', 6') se trouvent dans la position relative fermée ou ouverte, et présente une unité électronique de traitement de données,
**caractérisée en ce que**
l'unité électronique de traitement de données détecte l'intégrité structurelle et/ou fonctionnelle nécessaire à l'utilisation de la pince pour tuyaux souples (2 ; 2') de la pince pour tuyaux souples (2 ; 2') reçue dans le logement (42 ; 42') sur la base de la durée temporaire de la mesure effectuée par le capteur (26 ; 54) lors de la transition des sections de pince (4, 6 ; 4', 6') de la position relative fermée à la position relative ouverte ou de la position relative ouverte à la position relative fermée et la compare à un durée théorique temporaire de la mesure.

2. Pompe médicale (40 ; 40') selon la revendication 1,
**caractérisée par**
un actionneur (58) qui peut amener les sections (4, 6 ; 4' ; 6') de la pince pour tuyaux souples (2 ; 2') reçue dans le logement (42 ; 42') de la position relative fermée à la position relative ouverte et/ou de la position relative ouverte à la position relative fermée.

3. Pompe médicale (40 ; 40') selon la revendication 1 ou 2,
**caractérisée par**
une soupape de pompe (66) qui, en position fermée, recouvre le logement (42 ; 42') de sorte que la pince pour tuyaux souples (2 ; 2') ne puisse être retirée du logement (42 ; 42') ou ne puisse être introduite dans le logement (42 ; 42'), et libère le logement (42 ; 42') en position ouverte de sorte que la pince pour tuyaux souples (2 ; 2') puisse être retirée du logement (42 ; 42') ou puisse être introduite dans le logement (42 ; 42').

4. Pompe médicale (40 ; 40') selon les revendications 2 et 3,
**caractérisée en ce que**
l'actionneur (56, 58) peut amener les sections (4, 6 ; 4', 6') de la pince pour tuyaux souples (2 ; 2') reçue dans le logement (42 ; 42') en position fermée de la soupape de pompe (66) de la position relative fermée à la position relative ouverte et/ou de la position relative ouverte à la position relative fermée.

5. Pompe médicale (40 ; 40') selon l'une quelconque des revendications 2 à 4,
**caractérisée en ce que**
la position relative ouverte des sections (4, 6 ; 4', 6') de la pince pour tuyaux souples (2 ; 2') reçue dans le logement (42 ; 42') peut être modifiée par l'actionneur (56, 58) en sorte qu'un degré d'ouverture de l'intérieur du tuyau souple et donc un courant volumique de l'agent s'écoulant à travers le tuyau souple puissent être réglés.

6. Pompe médicale (40 ; 40') selon la revendication 5,
**caractérisée en ce que**
le capteur (26; 54) peut détecter la modification de la position relative ouverte des sections (4, 6 ; 4', 6') de la pince pour tuyaux souples (2 ; 2') reçue dans le logement (42 ; 42') de sorte que le degré d'ouverture de l'intérieur du tuyau souple et donc le courant volumique de l'agent s'écoulant à travers le tuyau souple puissent être détectés au moyen du capteur (26 ; 54).

7. Pompe médicale (40 ; 40') selon l'une quelconque des revendications 1 à 6,
**caractérisée en ce que**
le capteur (26 ; 54) détecte la position relative fermée des deux sections de pince (4, 6 ; 4', 6') en détectant la position d'une première (4 ; 4') des deux sections de pince (4, 6 ; 4', 6') et
le logement (42 ; 42') est conçu en sorte que la seconde (6 ; 6') des deux sections de pince (4, 6 ; 4', 6') puisse lui être fixée.

8. Pompe médicale (40 ; 40') selon les revendications 3 et 7,
**caractérisée en ce que**
la pompe (40 ; 40') présente un corps de base (64) vis-à-vis duquel la soupape de pompe (66) peut se déplacer,
le logement (42 ; 42') est disposé sur le corps de base (64) et
la première section de pince (4 ; 4') peut être déplacée au moyen de la soupape de pompe (66).

9. Pompe médicale (40') selon l'une quelconque des revendications 1 à 8,
**caractérisée en ce que**
le capteur (54) est un capteur de lumière (54) qui se déclenche lors d'une interruption, d'une modification et/ou d'un détournement d'un rayon lumineux émis par une source de lumière (52) agencée sur la pompe (40') à travers au moins l'une des sections de pince (4', 6').

10. Pompe médicale (40) selon l'une quelconque des revendications 1 à 8,
**caractérisée en ce que**
le capteur (26) est un capteur tactile (26), qui se déclenche au contact du capteur (26) avec au moins l'une des sections de pince (4, 6).

11. Pompe médicale (40) selon l'une quelconque des revendications 1 à 10,
**caractérisée en ce que**
il est prévu dans le logement (42') un bouton poussoir ou un cliquet (60) qui coopère avec adaptation de formes, dans la pince pour tuyaux souples (2') reçue dans le logement (42'), avec un logement de cliquet (62) formé dans une des sections de pince (6') pour assurer une fixation amovible.

12. Pince pour tuyaux souples (2') comprenant deux sections de pince (4', 6') mobiles l'une par rapport à l'autre, qui peuvent être amenées dans une position relative fermée l'une par rapport à l'autre, dans laquelle le tuyau souple agencé entre les sections de pince (4', 6') est pincé de manière à ce qu'aucun agent ne puisse s'écouler à travers l'intérieur du tuyau souple, qui peuvent être amenées en position relative ouverte l'une par rapport à l'autre, dans laquelle le tuyau souple agencé entre les sections de pince (4', 6') n'est pas pincé de sorte que l'agent puisse s'écouler à travers l'intérieur du tuyau souple et par lequel au moins une section de pince (6') peut être reliée à une pompe médicale (40 ; 40') selon l'une quelconque des revendications 1 à 11,
**caractérisée en ce que**
une première (4') des deux sections de pince (4', 6') présente une section qui recouvre un écran disposé sur la seconde (6') des sections de pince (4', 6') en position relative fermée des sections de pince (4', 6') et le libère au moins en partie ou ne le recouvre pas en position relative ouverte des sections de pince (4', 6').

13. Pince pour tuyaux souples (2) comprenant deux sections de pince (4, 6) mobiles l'une par rapport à l'autre, qui peuvent être pincées au moyen d'un élément d'arrêt (14) prévu sur la pince pour tuyaux souples (2) en position relative fermée, dans laquelle elles peuvent pincer un tuyau souple agencé entre elles de sorte qu'aucun agent ne puisse s'écouler à travers l'intérieur du tuyau souple et par lesquelles au moins une section de pince (6) puisse être raccordée à une pompe médicale (40) selon l'une quelconque des revendications 1 à 11,
**caractérisée en ce que,**
en plus de l'élément d'arrêt (14), il est prévu sur une première (4) des deux sections de pince (4, 6) une saillie (22) qui dépasse dans la direction de la seconde section de pince (6) en position relative fermée de sorte qu'elle s'étende jusqu'à un côté arrière opposé à la première section de pince (4) de la seconde section de pince (6) et que la position relative fermée des sections de pince (4, 6) de la pince pour tuyaux souples (2) reliée à la pompe médicale (40) puisse être détectée au moyen d'un capteur (26) agencé sur la pompe médicale (40) sur le côté arrière de la seconde section de pince (6).

14. Pince pour tuyaux souples (2) selon la revendication 13,
**caractérisée en ce que**
la saillie (22) s'étend en position relative fermée à travers un évidement usuel (24) dans la seconde section de pince (6).

15. Pince pour tuyaux souples (2) selon la revendication 13 ou 14,
**caractérisée en ce que**
les sections de pince (4, 6) peuvent être reliées l'une à l'autre à pivotement au moyen d'une charnière (8),
l'élément d'arrêt (14) est agencé respectivement sur le côté opposé à la charnière (8) de chacune des deux sections de pince (4, 6),
il est prévu un logement pour tuyau souple (30, 32) entre le côté de chaque section de pince (4, 6) tourné vers l'élément d'arrêt (14) et le côté tourné vers la charnière (8) et
l'évidement (24) et la saillie (22) sont agencés respectivement entre la charnière (8) et le logement de tuyau souple respectif (30 ; 32) de l'élément de pince correspondant (4 ; 6).

16. Système médical constitué d'une pompe médicale (40 ; 40') selon l'une quelconque des revendications 1 à 11 et d'une pince pour tuyaux souples (2 ; 2') selon l'une quelconque des revendications 12 à 15.
